# EUROPEAN PATENT APPLICATION

(11) **EP 4 112 123 A1**
(43) Date of publication of application: **04.01.2023**
(21) Application number: 21183524.4
(22) Date of filing: 02.07.2021
(51) Int. Cl.: A61N 5/10

(54) **PLANNING AND OPTIMIZATION OF MULTIMODALITY THERAPY**

(71) Applicant: RaySearch Laboratories AB, 104 30 Stockholm (SE)
(72) Inventor: Ödén, Jakob, SE-191 49 Sollentuna (SE); Eriksson, Kjell, 74651 Bålsta (SE); Fredriksson, Albin, 11869 Stockholm (SE); Traneus, Erik, 75239 Uppsala (SE)

(57) **Abstract**

A multimodality treatment plan including one or more of, for example, surgery, radiotherapy and a systemic treatment such as chemotherapy, may be obtained by robust co-optimization of two or more plans taking into account uncertainties affecting each treatment modality as well as uncertainties resulting from the combination of the modalities. Scenarios may be defined for the different uncertainties to be used in the optimization. The plans may be optimized simultaneously, or one plan may be optimized considering the predicted effect of another plan of another modality.

## Description

### Technical Field

The present invention relates to a method, a computer program product and a computer system for planning and optimization of multimodality treatments, for example, multimodality treatment of malignant diseases, such as cancer.

### Background

Multimodality therapy treatments involve using different types of therapy in combination for more effective treatment of a disease. The modalities often include two or more of the following: radiotherapy (RT), hyperthermia (HT), cryotherapy, chemotherapy, immunotherapy, hormonotherapy, and surgery, but may also include other local or systematic modalities.

Multimodality therapy treatments are commonly used for multiple malignant diseases. For example, cancer patients are often treated with surgery combined with chemotherapy, radiotherapy or both. The different treatments are often delivered sequentially, for example radiotherapy before or after surgery, but may also be delivered simultaneously for example hyperthermia combined with radiotherapy or chemotherapy, or intraoperative radiation therapy, where the radiotherapy treatment is delivered during surgery.

Currently in clinical practice, each modality in multimodality treatments is generally planned and optimized individually, which means that the combined effects of the different modalities, and how they may affect each other is not considered. Co-pending application EP21170669.2 relates to the simultaneous planning of hyperthermia therapy and radiotherapy treatment, taking into account the combined predicted effects of each plan.

### Summary of the invention

It is an object of the present invention to enable improved planning methods for multimodality treatment.

The invention relates to a computer-based method of planning a multimodality treatment including a first treatment plan of a first treatment modality and a second treatment plan of a second treatment modality, said method including the following steps:
- obtaining an optimization problem including an objective function related to the multimodality treatment,
- defining at least a first and a second scenario related to a first uncertainty in at least one parameter associated with the multimodality treatment,
- performing robust optimization of the optimization function value over the set of scenarios.

The present invention is based on the realization that the uncertainty in the total effect of the multimodality therapy treatment is affected by the individual modality uncertainties and the propagation of these in combination with other modality uncertainties. According to the present invention, uncertainties related to the multimodality treatment are accounted for already in the planning process. By utilizing robust optimization, the uncertainties are incorporated in the optimization problem. This means that their effect during optimization may be mitigated using various mathematical implementations. This is done to exploit the synergistic advantages and to avoid synergistic adverse effects, as well as to account for uncertainties originating from the individual modality treatments.

The at least one parameter may include a parameter related to the combined effect of the first and the second treatment plan. The at least one parameter may include one or more of the following:
- the spatial position of the treatment volume during the treatment,
- change of the spatial position of the treatment volume between the treatment modalities,
- the delivered quantities over the course of the treatment,
- any parameter affecting the treatment (e.g. heat transfer uncertainty of the hyperthermia treatment due to blood perfusion, tissue thermal and dielectric properties, the delivered specific absorption rate (SAR) distribution),
- the predicted combined effect of the different modalities,
- density of treated tissue,
- interplay effects of the various modalities,
- range uncertainty
- organ movement and/or biological model parameter values

The modalities to be used may include any combination of two or more of the following:
- radiotherapy,
- hyperthermia,
- chemotherapy,
- surgery,
- immunotherapy,
- hormonotherapy,
- cryotherapy
- any other therapy used in the treatment of malignant diseases

Particularly suitable combinations, which are frequently used in treatment of malignant conditions include:
- chemotherapy and radiotherapy
- chemotherapy and surgery
- chemotherapy and hyperthermia
- radiotherapy and surgery
- radiotherapy and hyperthermia

It should be noted that in each combination of treatment modalities above each of the modalities could be the first or the second treatment modality in the context of the plan. There could also be a third treatment plan, related to one of the first or second modalities, or to a third modality. For example, it is well known to use surgery, chemotherapy and radiotherapy together. More recent combinations include surgery, hyperthermia and radiotherapy or chemotherapy. Further treatment plans may also be included.

The step of selecting one or more uncertainties may involve selecting uncertainties that are specific to one or both of the two or more modalities and/or uncertainties that are a result of the combination of modalities.

In preferred embodiments, the first treatment plan has already been determined and step of calculating an optimization function value includes optimizing the optimization function value for the second treatment plan accounting for the pre-existing first plan. Alternatively, the step of calculating an optimization function value may include co-optimizing the first and the second plan by calculating the optimization function value accounting for the combined effect of the first and the second plan. One or more further plans can be added in a similar manner.

The optimization may include robust co-optimization of at least one plan of a modality and at least one plan of another modality. Alternatively, the optimization may include a robust optimization of at least one plan accounting for the total predicted effect of the multimodality treatment and treatment uncertainties of at least one pre-existing plan of another modality.

The treatment plans related to different modalities may be optimized for delivery simultaneously or on the same day, or on different days. If three or more plans are developed, some may be optimized for delivery on the same day and some for delivery on other days. For example, a hyperthermia treatment plan may be optimized for delivery together with a first radiotherapy treatment plan while a second radiotherapy treatment plan may be optimized for delivery on days when there is no hyperthermia treatment.

The at least one optimization function may comprise constraints which define parameters that are maintained during robust optimization of the objective function. Additionally, or alternatively, the at least one optimization function comprises a biological or a physical objective. Both objective functions and constraints, and their use in treatment optimization, are known per se.

The robust optimization may include machine parameter optimization of an arbitrary delivery system for one or more of the modalities constituting the multimodality therapy treatment. The optimization problem may include machine limitations as constraints and/or objective functions in a manner known per se.

In some embodiments, the robust optimization comprises one of the following:
- a stochastic programming approach, wherein the expected value of the objective function is minimized;
- a minimax approach, wherein the maximum of the objective function over the error scenarios is minimized;
- any combination of the two commonly referred to as minimax stochastic programming;
- a voxel-wise worst-case approach, in which the worst-case value to each voxel considered individually is optimized; or
- other types of robust optimization methods including value-at-risk or conditional-value-at-risk.

A therapeutic effect can be quantified using biological models comprising the equivalent radiation dose (EQD), equivalent uniform distribution (EUD), biological equivalent radiation dose (BED), thermal enhancement ratio (TER), tumor control probability (TCP), normal tissue complication probability (NTCP), complication free cure (CFC), secondary cancer (SC), cell survival fraction, and/or overall survival (OS).

According to some embodiments, the optimization problem comprises constraints which define parameters that are maintained during optimization. The use of constraints is well known in the art. The optimization problem may also comprises a biological or a physical objective.

In some embodiments, the optimization problem is defined to optimize machine parameters of at least one delivery system to be used in delivery of one of the plans as variables. Directly optimizing machine parameters eliminates the need for conversion to machine parameters after optimization. This may be, for example, be achieved by the optimization problem including at least one constraint and/or at least one objective related to machine limitations of at least one delivery machine that will be used to deliver hyperthermia treatment and/or radiation treatment as constraints or objectives.

The optimization problem suitably includes a simplified machine model for the delivery system or systems for which the parameters are optimized, such as radiation fluence optimization and power optimization for the heating system.

The present disclosure also relates to a computer program product comprising computer-readable code means which, when run in a computer, will cause the computer to perform the method according to any one of the embodiments discussed above. The computer program product is preferably stored on non-transitory storage medium.

The present disclosure also relates to a computer system comprising a processor and a program memory, the program memory holding a computer program product according to the above, to be executed in the processor.

### Acronyms

The following acronyms are used in this document:
BED - biological equivalent radiation dose
DVH - dose-volume histogram
EQD - equivalent radiation dose
EQD2 - equivalent radiation dose in 2-Gy fractions
EUD - equivalent uniform dose
HT - hyperthermia
LQ - linear-quadratic
NTCP - normal tissue complication probability
RT - radiotherapy
RTHT - thermoradiotherapy
TCP - tumor control probability
TER - thermal enhancement ratio

### Brief description of drawings

The invention will be described in more detail in the following, by way of examples and with reference to the appended drawings.
Figure 1 is a flow chart of a general method according to embodiments of the invention.
Figure 2 is a flow chart of a more specific method according to an embodiment of the invention.
Figure 3 is an overview of a computer system in which the present invention may be performed.

### Detailed description of embodiments

As explained above, many diseases are treated with a combination of two or more different types of treatment, or treatment modalities. For example, a cancer patient may be treated with a combination of one or more of surgery, chemotherapy, radiotherapy, hyperthermia, cryotherapy, hormone therapy and immunotherapy, depending on the type of cancer, and other factors. Each of these modalities is associated with uncertainties caused by factors that cannot be precisely known or controlled, such as the patient's exact position or movement, or responsiveness to a certain treatment.

According to the present disclosure, robust optimization methods are applied to multimodality treatment planning in such a way as to take into account uncertainties of both or all modalities individually as well as uncertainties regarding how they may affect each other in the treatment of the patient. Robust optimization of multimodality therapy treatments accounts for the combined effect and uncertainties of at least two different treatment modalities.

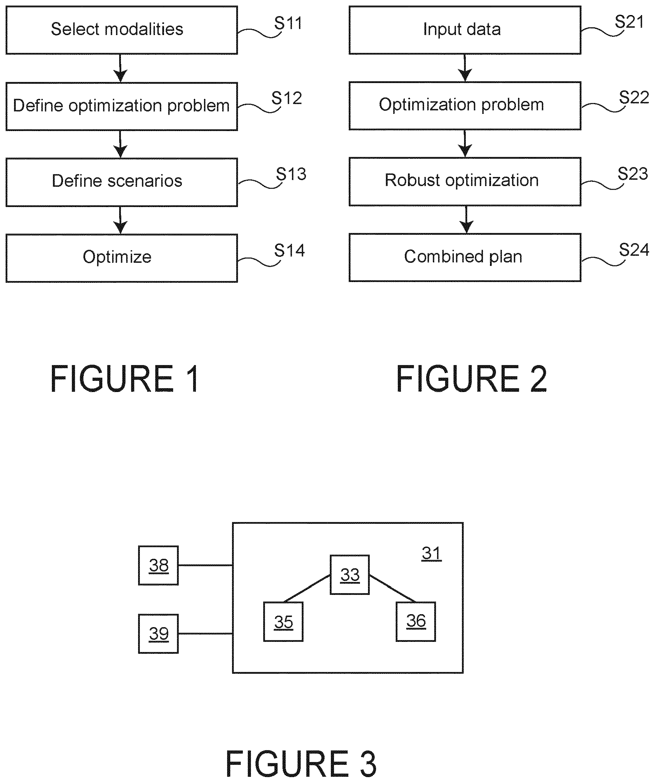

Figure 1 shows an overall flow chart of a method according to an embodiment of the invention for planning a multimodality treatment for a patient. In a first step S11, two or more treatment modalities to be used are selected. In a second step S12, an optimization problem is defined, including at least one optimization function based on a biological model taking the combined effect of the at least two treatment modalities into account. The model may include one or more of the following: the equivalent radiation dose - EQD, equivalent uniform distribution - EUD, biological effective dose - BED, thermal enhancement ratio - TER, tumor control probability - TCP, normal tissue complication probability - NTCP, complication free cure probability, secondary cancer, and/or overall survival.

In a third step S13, at least one uncertainty in the multimodality treatment, which may affect the outcome of the treatment, is determined and a set of scenarios covering different possible realizations of the at least one uncertainty is defined, and finally, in step S14, robust optimization is performed and evaluated over the set of scenarios.

The step of selecting one or more uncertainties may involve selecting uncertainties that are specific to each of the two or more modalities and uncertainties that are a result of the combination of modalities. Typical uncertainties relevant for the planning of the multimodality therapy treatment include the following:
- the spatial position of the treatment volume during the treatment,
- change of the spatial position of the treatment volume between the treatment modalities,
- the delivered quantities over the course of the treatment,
- any parameter affecting the treatment (e.g. heat transfer uncertainty of the hyperthermia treatment due to blood perfusion, tissue thermal and dielectric properties, the delivered specific absorption rate (SAR) distribution),
- the combined effect of the different modalities,
- density of treated tissue,
- interplay effects of the various modalities,
- organ movement
- for radiation therapy, range uncertainty regarding the position where the particle stops,
- biological model parameter values.

The optimization in step S14 may include either co-optimization of the two or more treatment plans of different modalities, or optimization of one treatment plan taking into account one or more pre-existing plans of one or more different modalities. In the former case, the step includes generating a multimodality plan by robust co-optimization of the optimization function value accounting for the combined effect of the at least two plans.

The optimization problem includes optimization functions, which typically includes at least one objective function related to a biological or a physical objective. Additionally, the optimization functions may include one or more physical and/or biological constraints which define parameters that are maintained during optimization.

According to this disclosure, robust optimization functions are used, which are based on the (biological) effect of the multimodality therapy treatment accounting for uncertainties of the included modalities using scenarios of specific realization of uncertainties relevant for multimodality treatment. The effect of the multimodality treatment may be quantified through mathematical expressions of an equivalent quantity of interest, e.g the equivalent radiation dose (EQD), and/or any therapeutic effect quantified using biological models comprising equivalent uniform distribution (EUD), biological equivalent radiation dose (BED), thermal enhancement ratio (TER), tumor control probability (TCP), normal tissue complication probability (NTCP), complication free cure (CFC), secondary cancer (SC), cell survival fraction, and/or overall survival (OS).

The uncertainties to be considered are different for different modalities. Also, different combinations of modalities lead to different uncertainties in how they may affect each other. For chemotherapy, immunotherapy and hormonotherapy, uncertainties in the biological effect of the treatment may be caused by uncertainties in the uptake of the drug, distribution of the drug, the oxygenation of the tissue etc. For surgery there are uncertainties in whether or not all malignant cells in the volume of interest have been successfully removed, because of uncertainties in the extent of the tumor, and potential microscopic spread of malignant cells beyond the solid tumor which require a large resection margin. With an increased margin of resection, the NTCP increases as the likelihood of damaging surrounding structures increases.

As an example, typical uncertainties that may be considered when planning a combined hyperthermia and radiotherapy treatment will be listed in the following:
Uncertainties in HT treatments typically include, for example
   - Uncertainties in the patient position or anatomy during the treatment
   - Uncertainties in something affecting the heat transfer (e.g., blood perfusion, tissue thermal and dielectric properties, the delivered specific absorption rate (SAR) distribution)
   - Uncertainties in the temperature delivery over the HT treatment (i.e., in the thermal dose)
   - Uncertainties in the amount of the direct cell kill
Uncertainties in the RT treatment may include, for example:
   - Uncertainties in the patient position or anatomy during the treatment
   - Uncertainties in tissue composition and density
   - Uncertainties due to organ motion due to e.g., breathing, various organ content etc.
   - Range uncertainty regarding the position where the particle stops
Uncertainties caused by the combination of two modalities in RTHT treatment may include
   - Uncertainties in the temperature dependency of the radio-sensitization due to inhibition of DNA repair, reoxygenation etc.
   - Uncertainties in the change of the patient position or anatomy between the hyperthermia treatment and the radiation therapy treatment
   - Uncertainties in the calculation of the combined biological effect of the RTHT treatment

These uncertainties can then be considering using different methods in the treatment plan optimization process, for example one or more of the following:
- a scenario-based method using multiple temperature distributions resulting from different realizations of the uncertainties of the HT treatment
- worst case optimization over scenarios
- expected value optimization over scenarios
- other types of robust optimization, such as CVaR, minimax stochastic an others
- optimization using at least one aggregate temperature distribution resulting from the scenarios, for example voxel-wise max and min temperature over the scenarios
- using a single temperature distribution aggregating the effects of the uncertainties, for example, smearing of the temperature distribution to expand high or low temperatures
- a scenario-based method using a single temperature distribution but multiple realizations of the uncertainties during the RT treatment.

Hence, optimization of an HTRT plan may be performed, by taking into account the predicted effect of a pre-existing HR or RT plan when optimizing the other plan, that is, the RT or HT plan, respectively. This may be done, for example according to the following, as shown in Figure 2, assuming that the HT plan is pre-existing: Input data S21 includes the predicted temperature distribution resulting from this pre-existing hyperthermia plan. An optimization problem S22 is also obtained, including robust optimization functions where the temperature dependence of the biological parameters with uncertainties are included for each voxel of interest. The input data S21 is used as input for the robust optimization S23 of a radiotherapy plan, using the optimization problem S22 accounting for uncertainties in the combined RTHT treatment (including uncertainties originating from HT and/or RT treatments). As a result an optimized multimodality treatment comprising the hyperthermia plan and the newly optimized radiotherapy plan is obtained, S24. This procedure may easily be adapted for other combinations of modalities.

The optimization problem S22 may be defined according to principles that are known to the skilled person. For example, the EQD in 2-Gy fractions (EQD2) of the combined treatment could be optimized using the linear-quadratic (LQ) model for cell survival. The LQ-parameters α and β are known to vary with temperature, as an increased temperature causes an increase in the radiosensitivity. By incorporating models for the temperature dependency of the LQ-parameter it is possible to optimize directly on the EQD2 for the combined effect of radiation dose and temperature. Furthermore, the EQD2 could be incorporated in e.g., TCP and/or NTCP models for optimization. In this manner the temperature dependent EQD2, TCP and/or NTCP may be optimized to fulfill the stated clinical goals.

As an alternative to the process shown in Figure 2, for example, a pre-existing dose distribution of a radiotherapy plan may be used as input for the robust optimization of a hyperthermia plan accounting for uncertainties in the combined RTHT treatment (including uncertainties originating from HT and/or RT treatments). The objective function comprises robust optimization functions where the effect of the radiation dose is included for each voxel of interest. As above, optimization strategies using e.g., EQD2, TCP, and NTCP models could be used for the optimization of the hyperthermia plan.

Another common combination of treatment modality is surgery and radiotherapy. In some cases, radiotherapy is used before surgery, to shrink the tumor so that it can be more easily removed. In other cases, radiotherapy is used after surgery, to treat the area surrounding the tumor in case of any remaining cancerous cells. The following example discusses the robust optimization of a multimodality therapy treatment consisting of a RT plan and a surgery plan.

In the case of a pre-existing RT-plan, the surgery could be optimized to ensure removal of any malignant cell left after radiation, accounting for surgery specific uncertainties stated above, as well as uncertainties in the effect of the RT treatment due to RT specific uncertainties mentioned above.

In case of a pre-existing surgery plan, the RT plan can be robustly optimized to eradicate all malignant cells accounting for RT specific uncertainties such as setup and particle range, as well as for the uncertainties in e.g. the surgical removal of malignant cells at the tumor borders and the NTCP of organs depending on the surgical removal margin. For a parallel-structured organ like the liver, the NTCP depends strongly on the removed organ volume, whereas other functions are directly connected to the sparing of e.g., nerve paths. A pre-existing plan of a surgery is used as input for the robust optimization of a radiotherapy plan accounting for uncertainties in the combined RT+surgery treatment (including uncertainties originating from surgery and/or RT treatments).

In this case, the objective function comprises robust optimization functions where the surgery dependence of the biological parameters with uncertainties are included for each voxel of interest. For example, the EQD2 of the combined treatment could be optimized using the LQ model for cell survival. By incorporate models of the surgical removal of malignant cells, one could optimize directly on the EQD2 for the combined effect of radiation dose and surgery to ensure eradication of the malignant cells. Furthermore, the EQD2 could be incorporated in e.g., TCP and/or NTCP models for optimization. In this manner one optimizes the EQD2, TCP and/or NTCP to fulfill the stated clinical goals for the RT+surgery treatment.

In the following, two examples of co-optimization will be given, that is, simultaneous optimization of two treatment plans of different modalities.

For co-optimizing a RTHT treatment plan, the temperature and dose distributions of a hyperthermia and a radiotherapy plan, respectively, are robustly co-optimized accounting for uncertainties in the combined RTHT treatment, including uncertainties originating from HT and/or RT treatments. The optimization problem comprises robust optimization functions where the combined effect of temperature and dose are included for each voxel of interest. As in examples above, optimization strategies using e.g., EQD2, TCP, and NTCP models could be used, however, in this example the temperature and dose distributions are robustly co-optimized in the search for the optimal treatment.

Alternatively, the multimodality treatment could include surgery and RT treatment, there the surgery plan and RT plan are optimized simultaneously accounting for uncertainties of both modalities. A pre-existing dose distribution of a radiotherapy plan may be used in this case as input for the robust optimization of a surgery plan accounting for uncertainties in the combined RT+surgery treatment, including uncertainties originating from surgery and/or RT treatments. The optimization problem comprises robust optimization functions where the combined effect of surgery and radiation dose are included for each voxel of interest. The plan of the surgery and the dose distribution of a radiotherapy plan are robustly co-optimized accounting for uncertainties in the combined RT+surgery treatment (including uncertainties originating from surgery and/or RT treatments). As in examples above, optimization strategies using e.g., EQD2, TCP, and NTCP models could be used, however, in this example the surgery and dose distributions are robustly co-optimized in the search for the optimal treatment.

In general, in the context of the present invention, the optimization function may include a simplified machine model for the system of the modalities for which the parameters are optimized, such as radiation fluence optimization for a radiation system and power optimization for the heating system. The physical objective or objectives are selected in dependence of the treatment modalities and typically comprise radiation dose, thermal dose, drug uptake limits to targets and organs at risk (OAR) in the treatment volume, volume histogram limits, linear energy transfer (LET) limits for radiotherapy, the location where the particles stop and/or homogeneity and conformity indices. For surgery, physical objectives may include surgical resection volumes, degree of overlap between tumor and risk organ, and cut surfaces and/or planes.

Figure 3 illustrates schematically a computer 31 in which methods according to the invention may be performed. The computer 31 comprises a processor 33, connected to a program memory 35 arranged to hold one or more computer programs that are to be run in the processor. The computer also comprises a data memory 36 arranged to hold data to be used in the execution of the programs, and data resulting from the execution. As is common in the art a number of input and output units 38, 39 are typically connected to the computer 31, including a screen, a keyboard and other suitable units. As will be understood, the program memory and/or the data memory may be implemented as one or several memory units in or outside of the computer 31, for example in the cloud. In the present invention the data memory may be arranged to hold input data such as patient data, typically including histological data about the different tissues. The data memory 36 may also hold information about the different scenarios, information about the set of plans and of the previously obtained plans used as input to the optimization. The data memory 36 may also hold the resulting set of plans that forms the output from the method.

## Claims

1. A computer-based method of planning a multimodality treatment including a first treatment plan of a first treatment modality and a second treatment plan of a second treatment modality, said method including the following steps:
- obtaining an optimization problem including an objective function related to the multimodality treatment,
- defining at least a first and a second scenario related to a first uncertainty in at least one parameter associated with the multimodality treatment,
- performing robust optimization over the set of scenarios.

2. A method according to claim 1, wherein the multimodality treatment includes treatment plans for two or more of the following treatment modalities:
- radiotherapy
- chemotherapy
- surgery
- hyperthermia
- immunotherapy
- cryotherapy
- hormonotherapy

3. A method according to any one of the preceding claims, wherein the at least one parameter includes a parameter related to the predicted combined effect of the first and the second treatment plan.

4. A method according to any one of the preceding claims, wherein parameters includes one or more of the following:
- the spatial position of the treatment volume during the treatment,
- change of the spatial position of the treatment volume between the treatment modalities,
- the delivered quantities over the course of the treatment,
- any parameter affecting the treatment (e.g. heat transfer uncertainty of the hyperthermia treatment due to blood perfusion, tissue thermal and dielectric properties, the delivered specific absorption rate (SAR) distribution),
- the combined effect of the different modalities,
- density of treated tissue,
- interplay effects of the various modalities,
- range uncertainty,
- organ movement and/or

5. biological model parameter values.A method according to any one of the preceding claims, wherein the first treatment plan has already been determined and the robust optimization involves optimizing the optimization function value for the second treatment plan accounting for the pre-existing first plan.

6. A method according to any one of the claims 1 - 5, wherein the robust optimization includes co-optimizing the first and the second plan by calculating the optimization function value accounting for the combined effect of the first and the second plan.

7. A method according to any one of the preceding claims, wherein the first treatment plan is a chemotherapy treatment plan, a surgery treatment plan or a hyperthermia treatment plan and the second treatment plan is a radiotherapy treatment plan.

8. A method according to any one of the preceding claims, wherein the first treatment plan is a radiotherapy treatment plan and the second treatment plan is a surgery treatment plan, a chemotherapy treatment plan or a hyperthermia treatment plan.

9. A method according to any one of the preceding claims, wherein the multimodality treatment plan further includes one or more additional treatment plans.

10. A method according to any one of the preceding claims, wherein the optimization problem comprises constraints which define parameters that are maintained during optimization.

11. A method according to any one of the preceding claims, wherein the optimization problem comprises a biological or a physical objective.

12. A method according to any one of the preceding claims, wherein the optimization problem is defined to optimize machine parameters of at least one delivery system arranged to deliver one of the treatment plans. as variables, for example.

13. A method according to claim 12, wherein the optimization problem includes a simplified machine model for the delivery system.

14. A computer program product comprising computer-readable code means which, when run in a computer, will cause the computer to perform the method according to any one of the preceding claims.

15. A computer system comprising a processor and a program memory, the program memory holding a computer program product according to claim 14 to be executed in the processor.
